# EUROPEAN PATENT APPLICATION

(11) **EP 3 904 874 A1**
(43) Date of publication of application: **03.11.2021**
(21) Application number: 20171921.8
(22) Date of filing: 28.04.2020
(51) Int. Cl.: G01N 33/38, G01N 25/48, G01N 23/207, G01N 33/24

(54) **METHOD FOR QUANTIFYING THE PHASE AMOUNTS OF COMPONENTS OF A CARBONATED ROCK**

(30) Priority: 27.04.2020 PT 2020020739
(71) Applicant: Universidade de Évora, 7000-803 Évora (PT)
(72) Inventor: PAULO MIRÃO, José António, 7000-019 N Senhora Graça do Divor (PT); MARTINS MOITA, Patrícia Sofia, 7005-482 Évora (PT)
(74) Representative: Patentree

(57) **Abstract**

The present disclosure relates to a method for quantifying the phase amounts of a carbonated rock sample comprising the steps of: calibrating a differential thermal analysis, DTA, line from a plurality of quartz references in varying amounts of quartz relative to the total reference amount, with the amount of quartz as a function of the area of endothermic peak quartz inversion; obtaining a DTA area of endothermic peak inversion of the quartz in the sample and calculating the amount of quartz present in the sample using the calibrated DTA line; obtaining an X-ray diffraction, XRD, pattern from the sample; using Rietveld Refinement to fit XRD patterns of crystalline carbonated rock components to the obtained sample XRD pattern, provided that the amount of quartz present in the sample is fixed to be the previously calculated amount; calculating the amounts of the crystalline rock components, other than quartz, from the fitted XRD patterns; calculating a total amount of amorphous and minor phases present in the sample by subtracting the sum of the calculated amounts of the crystalline rock components, including quartz, from the total sample amount.

## Description

### TECHNICAL FIELD

The present disclosure relates to a method to quantify the mineralogical phases present in a carbonate rock like a marble, a limestone or a marl. The present disclosure can be applied in the presence of quartz, used as internal standard in Rietveld refinement. The present disclosure may be applied to the cement and the dimension stone industry and the building conservation-restoration community.

### BACKGROUND

Limestone, marls and marble are carbonated rocks with a mineralogical composition comprising carbonates (e.g. calcite) and other associated phases, in variable amounts, such as quartz, iron oxides or clay minerals.

Those in carbonated rocks are commercially exploited in the industry of cement (or lime) and dimensional stone. To ensure quality control of these kind of rocks, the industrial transformation requires the quantification of each mineral phase.

Modern cements (i.e. Portland cement) is used to bind aggregates as sand or gravel in a concrete which is the more important building material in modern construction industry.

The method of production of cement, using limestone or marl, is a controlled chemical process that combine calcium, aluminium, iron and silicon.

From this chemical process results new phases as C₃S (Alite - 3CaO·SiO₂), C₂S (Belite - 2CaO.SiO₂), C₃A (Tricalcium aluminate - 3CaO·Al₂O₃), C₄AF (Brownmillerite - 4CaO·Al₂O₃-Fe₂O₃).

The capacity of the cement to bind depends on its chemical and phase composition.

The chemical and phase composition of the cement is a consequence of the chemical process and chemical and mineralogical composition of the raw materials.

Calcite in limestone is the main source of calcium; but the presence of other material like clays and quartz is essential to get the correct phase ratios. Therefore, the cement industry needs to control careful the raw materials used.

Lime mortar widely used in built heritage is a combination of lime binder (carbonated to calcite nowadays) and siliceous sand (mainly quartz).

Traditional lime, that is a main component of lime mortars, results from the calcination of limestone.

During mortars conservation-restoration works, the new mortars recipe must follow the historical ones, being physical and chemical compatible.

To achieve these requirements, it is essential to know the mineralogical composition of the historical mortars.

The dimension stone industry is a global business where limestone and marble are the more important commercialized rocks.

Their characteristics as durability, mechanical properties and response to different surface treatments (e.g. polishing) depends on their texture and mineralogical composition.

Although calcite is always the predominant mineral phase, the common presence of quartz and clay minerals is usually a devaluing factor in limestone and marble dimension stones markets.

The quantification of the amount of the different mineralogical phases in cement raw materials, in lime mortars from building heritage and dimensional stone is mandatory.

Conventionally, the quantification of mineral phases in cement raw materials, historical mortars and dimension stones is done by Thermal Gravimetric Analysis (TGA) or Rietveld refinement after X-ray Diffraction (XRD).

The method of TGA is based on the measurement of the sample mass during heating.

The TGA method quantifies the phases according to the mass loss of the components such as carbonates.

Due to carbonates thermal decomposition, CO₂ is release and their proportion can be calculated in the sample.

The TGA results can be complex because it is not straightforward assign a mass loss to the thermal decomposition of a specific mineral.

X-ray diffraction (XRD) is an analytical technique primarily used for phase identification of a crystalline material. Over this technique, the Rietveld refinement (Rietveld, 1969) allows phases quantification. XRD is a low-sensitivity method to detect low-concentration phases, however is unable to identify amorphous or low-crystallinity phases as natural iron oxides or some clay minerals.

Differential thermal analyses (DTA) is a technique in which the difference in temperature between the sample and a reference material is monitored against time or temperature, while the sample is subject to a specific temperature and atmosphere (International Confederation for Thermal Analysis).

The reference material should be a material without any endothermic or exothermic reaction in the temperature range of the experiment.

The most suitable crucibles are the Pt-Ir; they resist to high temperatures (1500ºC) and do not react with the analysed materials at the temperature of interest.

The type of atmosphere, in the chamber, for the experiment can change between several gases (e.g. air, N₂, CO₂, He, Ar) used at a constant flux. For a TGA-DTA equipment N₂ is the suitable gas.

The temperature range for the analysis must include the intervals at which the processes to be studied occur. The well-known quartz-a to quartz-β displacive phase transition or inversion and it is accompanied by a linear thermal expansion of 0,45%. The processes of α-β transition involves a symmetry change from low temperature α-structure, space group P3₂21 to high temperature space group P6₄22 of the β-phase (Peng & Redfern, 2013). From quartz-a to quartz-β, the process is strongly endothermic with a DTA peak a 573,3ºC (Dolino, 1990).

The shape of DTA peak obtained in the previous paragraph depends on the amount of quartz, grain size distribution of specimen and heating rate (e.g. Earnest et al., 2018); higher heating rate → higher DTA peak, higher amount of quartz → higher DTA peak, less variation in grain size → narrow DTA peak.

Typical heating rate for operation is 10°C/min and for sample mass a chosen value depends on the capacity of the crucible (e.g. 25-50mg).

DTA was used to quantify quartz (Grimshaw, 1953; Sheffield, 1994) in complex samples as soils (Karathanasis & Harris, 1994) or ceramics (Earnest et al, 2018). The method shows to be more precise than X-Ray diffraction (Rowse & Jepson, 1972) and dependent of the particle size (Weiss et al, 1970).

These facts are disclosed in order to illustrate the technical problem addressed by the present disclosure.

### GENERAL DESCRIPTION

The present disclosure is a new method of carbonated materials (limestone, marl, marble or lime mortars) phase quantification in the presence of quartz.

The analytical procedure is based in the combination of Differential thermal analysis (DTA) and Rietveld refinement (Rietveld, 1969) after X-ray diffraction (XRD).

By DTA, the presence of quartz would be identified and quantified.

The quartz is used as internal standard in the XRD Rietveld quantification; the minor phases and the amorphous phases can now be quantified.

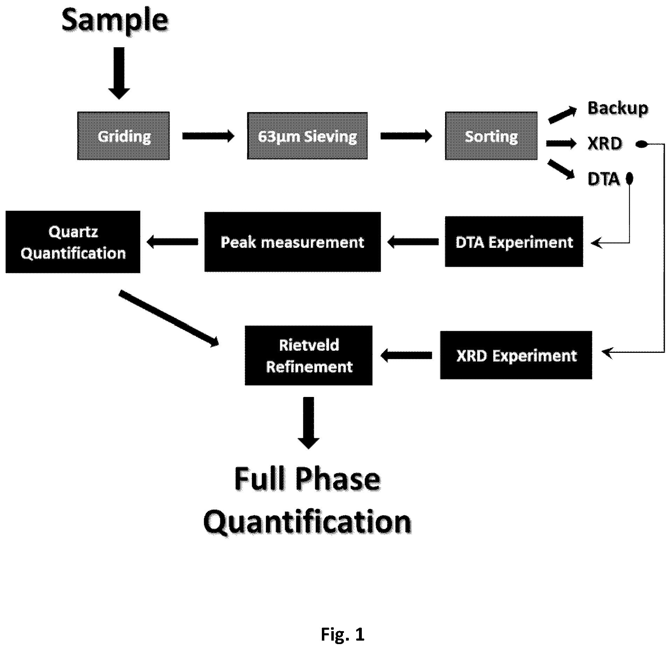

The method is split in three steps (figure 1).

First step includes the preparation of the sample and calibration of the Differential thermal analysis instrument.

In the Second step, after [0038], DTA can be carried out; the amount of quartz would be quantified considering the endothermic peak quartz inversion at 573°C and the equipment calibration.

Third, after [0038], the XRD experiment can be carried out. Finally, after quartz quantification on [0039] Rietveld refinement produce the final quantification of the phases in the carbonated materials.

The proposed methodology accomplishes the full quantification of the phases present, including minor and amorphous phases, in limestones and marls cement raw materials, lime mortars and dimension limestone or marbles.

The final phase composition of cement is already controlled by XRD; therefore, for the companies, only the acquisition of DTA equipment is required.

The present disclosure relates to a method for quantifying the phase amounts of a carbonated rock sample comprising the steps of:
calibrating a differential thermal analysis, DTA, line from a plurality of quartz references in varying amounts of quartz relative to the total reference amount, with the amount of quartz as a function of the area of endothermic peak quartz inversion;
obtaining a DTA area of endothermic peak inversion of the quartz in the sample and calculating the amount of quartz present in the sample using the calibrated DTA line;
obtaining an X-ray diffraction, XRD, pattern from the sample;
using Rietveld Refinement to fit XRD patterns of crystalline carbonated rock components to the obtained sample XRD pattern, provided that the amount of quartz present in the sample is fixed to be the previously calculated amount;
calculating the amounts of the crystalline rock components, other than quartz, from the fitted XRD patterns;
calculating a total amount of amorphous and minor phases present in the sample by subtracting the sum of the calculated amounts of the crystalline rock components, including quartz, from the total sample amount.

In an embodiment, the DTA area of endothermic peak inversion is obtained at a temperature ranging from 40° to 800°C, with a step heating of 10 °C/min.

In an embodiment, the DTA area of endothermic peak inversion is obtained at a temperature of 573°C.

In an embodiment, the DTA area of endothermic peak inversion is obtained under nitrogen atmosphere at a flow rate of 70 mL/min.

In an embodiment, the XRD pattern is obtained at 0.02°steps for 4 seconds or more by step.

In an embodiment, the XRD pattern obtained comprise diffraction peaks from atomic planes with d=17Å to d=1,3Å.

In an embodiment, the instrument Rietveld parameters are obtained by fitting corundum (Al₂O₃) reference.

In an embodiment, the Rietveld parameters to fit XRD patterns of crystalline carbonated rock are Instrument/sample alignment instrumental parameters, preferred orientation, grain size and strain-stress characteristics of grains, the crystal structure parameters and the proportions of each mineral phase.

In an embodiment, the calibration of DTA is made in the same conditions of the DTA to obtain the area of endothermic peak inversion of the sample.

In an embodiment, the plurality of quartz references used for calibrating a Differential Thermal Analysis, DTA, comprise a variable amount of calcite euhedral crystals to make up the total reference amount.

In an embodiment, the variable amount of calcite euhedral crystals in the plurality of quartz references are in steps proportions of 5%.

In an embodiment, the plurality of quartz references have particles size of 63 µm or less.

In an embodiment, the carbonated rock sample is ground to obtain particles size of 63 µm or less and the rock sample after ground is quartered for the differential thermal analysis, DTA, and for X-ray diffraction, XRD.

In an embodiment, the carbonate rock is a marble, a limestone, lime mortar or a marl.

It is also disclosed a non-transitory storage media comprising computer program instructions for implementing a device for quantifying the phase amounts of a carbonated rock sample, the computer program instructions including instructions which, when executed by a processor, cause the processor to carry out the disclosed method.

A new method of phase quantification in lime mortars, marls, limestones and marble is proposed. It is expected that the disclosure can be successfully applied in dimension stone and cement industry and by the building conservation community.

Quartz and calcite euhedral crystals may be milled to get particles with less than 63 µm.

The quartz and the calcite powders may be mixed in several proportions (see table 1), in steps of 5%, from 100% of calcite to 100% of quartz.

The different combinations from [0060] may be used as standards in DTA (i.e. Differential thermal analysis) experiments.

The experimental conditions may be close of: from room temperature (e.g. 40ºC) until 800ºC with a step heating of 10°C/min, under nitrogen atmosphere with a flow rate of 70 mL/min.

The area of endothermic peak quartz inversion at 573°C, in each experiment, may be measured.

A calibration line that relate the amount of quartz from [0060] with area of endothermic peak quartz inversion from [0063] may be create.

Each limestone, marl, marble or lime mortar sample may be milled to obtain a powder with less than 63µm that is subsampled to XRD (i.e. X-ray diffraction) and DTA experiments.

The DTA experiment may run with experimental conditions close to: from room temperature (e.g. 40°C) until 800°C with a step heating of 10ºC/min, under nitrogen atmosphere with a flow rate of 70 mL/min.

The area of the endothermic peak quartz inversion may be measured and the amount of quartz obtained considering the calibration line from [0064].

The XRD instrument Rietveld parameters may be obtained by fitting an Al₂O₃ standard. These parameters may be used in the next fitting procedures.

The XRD experiments in the limestone, marl, marble or lime mortar samples using the following experimental conditions: 0.02º steps, not less than 4 seconds by steep and from atomic planes with d=17Å to atomic planes with d=1,3Å.

After phase identification, the quartz peaks may be fitted using the Rietveld approach and considering the refinements of the Instrument/sample alignment instrumental parameters, the preferred orientation, grain size and strain-stress characteristics of grains and the crystal structure parameters. The crystal structure parameters are constrained and cannot change more, from the initial values, than 5%.

The amount of quartz in the sample may be fixed using the values obtained by DTA (see [0067]) and the other identified phases peaks may be fitted as quartz in [0070].

The position, intensity and shape of the diffraction peaks may be fitted in order to obtain the characteristics of others phases than quartz and their abundance.

The amount of each crystalline phase is obtained considering [0067] and [0072].

The total amount of minor phases, not detected by XRD, and amorphous phases may be the difference between 100% and the sum of the crystalline phases obtained in [0073].

### BRIEF DESCRIPTION OF THE DRAWINGS

The following figures provide preferred embodiments for illustrating the disclosure and should not be seen as limiting the scope of invention.
**Figure 1****:** Schematic representation of the different steps and tasks of the methodology to marl, limestone and marble phase quantification in the presence of quartz.

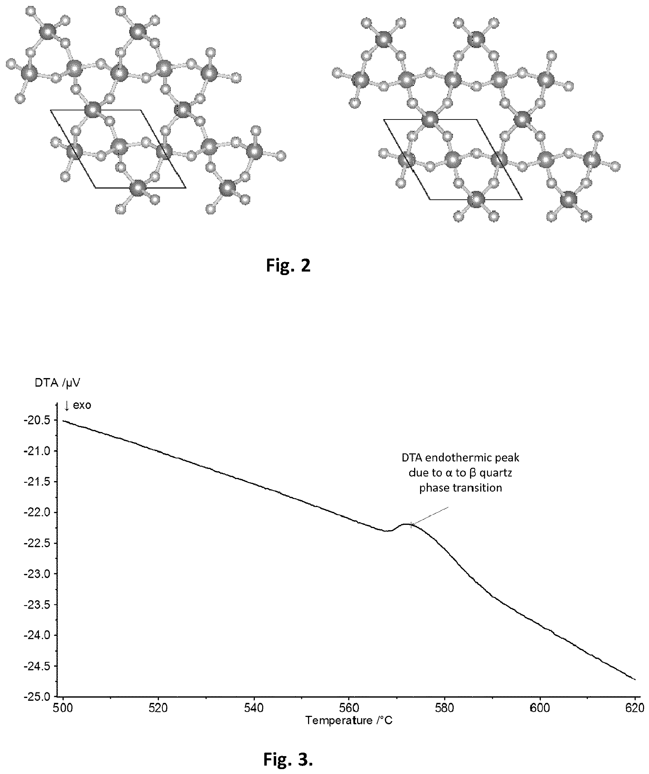

**Figure 2****:** Ball-and-stick representation of the quartz-a (left) and quartz-β (right) after the data of Kihara, K. (1990).
**Figure 3****:** Representation of DTA endothermic peak due to quartz-a to quartz-β phase transition.

### DETAILED DESCRIPTION

The present disclosure describes a new methodology to the full quantification of the phases present in limestones and marls used as raw materials in ceramic industry, in dimension stones and in lime mortars.

The method involves the following steps:
(a) sample preparation,
(b) Differential thermal analysis (DTA) and
(c) Rietveld refinement after the X-ray diffraction (XRD) experiments.

The procedure requires an X-ray diffractometer, a DTA instrument and general laboratory equipment to the sample preparation.

Previously, the DTA instruments must be calibrate in order to assure a guaranteed relation between the endothermic peak of the quartz and the amount of this mineral in the sample.

For calibration of the DTA instrument, samples of quartz and a calcite euhedral crystal are required.

The calcite and the quartz crystal are ground manually or mechanically.

Each resulted powder from [0084] is sieved by 63µm sieve. The material with less than 63µm is kept. For the material with more than 63µm, the gridding is repeated until all the calcite and the quartz have granulometry lower than 63µm.

To design a calibration line, several combinations (Tab. 1) of different proportions of pure calcite and quartz euhedral phases [taken from [0085] are heated until the temperature of interest (800°C) (see [0028]).

**Table 1 - Considered proportions of pure calcite and quartz euhedral phases for the design of calibration line.**

| | wt% | wt% | wt% | wt% | wt% | wt% | wt% | wt% | wt% | wt% | wt% |
|---|---|---|---|---|---|---|---|---|---|---|---|
| quartz | 0 | 5 | 10 | 15 | 20 | 30 | 40 | 50 | 80 | 95 | 100 |
| calcite | 100 | 95 | 90 | 85 | 80 | 70 | 60 | 50 | 20 | 5 | 0 |

Each proportion calcite/quartz is taken as specimen, and heated in a DTA instrument with the following conditions: from room temperature (e.g. 40°C) until 800°C with a step heating of 10°C/min, under nitrogen atmosphere with a flow rate of 70 mL/min.

For each DTA signal the area of the endothermic peak (chart DTA vs Temperature) of the α to β quartz phase transition is measured following the definition of the baseline. Most instruments handle with processing options that can be used to construct the baseline and calculate the peak area.

Using the data from table 1, a calibration line that related the amount of quartz and area of endothermic peak is built. Each calibration line is only valid for a specific instrument.

Each sample of cement raw material, dimension stone or lime mortar is ground following the procedures of [0084] and [0085]to obtain particles lower than 63µm. The sample will be sub-sampled by the coning and quartering method until suitable samples for DTA and XRD are obtained.

The DTA will be conducted in the same conditions as those used for design of calibration line in [0088]; from room temperature (e.g. 40°C) until 800°C with a step heating of 10°C/min, under nitrogen atmosphere with a flow rate of 70 mL/min.

The area of the endothermic peak due to quartz α to β phase transition is measured. The measured value is converted in the mass of quartz in the sample using the calibration line [0090].

The XRD instrument Rietveld parameters must be obtained in what concern the Instrumental Parameters as: Radiation (spectral purity and Lorentz Polarization), Instrument/sample alignment, Axial divergence of the beam, Geometry and configuration and Beam Conditioning. An Al₂O₃ (mineral corundum) standard is required. The standard runs in the same condition of the samples [0095]. The Rietveld fitting of the corundum pattern is done considering the structural data of Kirfel & Eichhorn (1990) and allow the refinement of the Instrumental Parameters. The Instrumental Parameters are used in following experiments.

The XRD experiment must be conducted in a reliable instrument. The scan must be collected with 0.02º steps, not less than 4 seconds by step and in order to obtains diffraction peaks from atomic planes with d=17Å to d=1,3Å.

The phases should be identified using the software that accomplish the XRD instrument or employing freeware databases and software.

The peaks of quartz can now be fitted allowing the refinement of Instrument/sample alignment instrumental parameters, crystal structure parameters and the preferred orientation, grain size and strain-stress of the structure. The initial crystal structure parameters are taken from Kihara (1990). The refinement of crystal structure parameters is allowed but the changes are constrained by 5% of the initial values.

The amount of quartz in the sample is fixed using the values obtained in [0093] by DTA.

The others [0096] identified phases are fitted. During the first run, the crystal structure parameters that affects the peak position (i.e. Unit cell parameters) are fitted. The peak shape is fitted in second run, adding the atomic parameter, the preferred orientation (especially important in clays) and absorption. Finally, in the last run, the peak shape is considered by adding the specimen properties grain size and the strain-stress and the crystal structure components as crystallite and disorder of the major phases.

During all the process, the abundance of quartz is fixed by the values obtained in DTA [0098] and the abundance of other phases is allowed to change. Its final value is register in the final run as the abundance of the different phases obtained by XRD Rietveld refinement using quartz as internal standard.

When the abundance of all is inferior to 100%, the value x=100-sum must be assigned to amorphous or minor phases not identified by XRD.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof.

The following claims further set out particular embodiments of the disclosure.

### REFERENCES

Dolino, G. (1990). The α-inc-β transitions of quartz: a century of research on displacive phase transitions. Phase Transitions: A Multinational Journal, 21(1), 59-72.
Earnest, C. M., Cassel, R. B., & McCurdy, J. (2018). Studying clay formulations for porcelain ceramics using simultaneous thermal analysis (STA) techniques. Ceramic Sciences and Engineering.
Grimshaw, R. W. (1953). The quantitative estimation of silica minerals. Clay Minerals Bulletin, 2(9), 2-7.
Karathanasis, A. D., & Harris, W. G. (1994). Quantitative thermal analysis of soil materials. Quantitative methods in soil mineralogy, 360-411.
Kihara, K. (1990). An X-ray study of the temperature dependence of the quartz structure. European Journal of Mineralogy, 63-78.
Kirfel, A., & Eichhorn, K. (1990). Accurate structure analysis with synchrotron radiation. The electron density in Al2O3 and Cu2O. Acta Crystallographica Section A: Foundations of Crystallography, 46(4), 271-284.
Peng, Z., & Redfern, S. A. (2013). Mechanical properties of quartz at the α-β phase transition: Implications for tectonic and seismic anomalies. Geochemistry, Geophysics, Geosystems, 14(1), 18-28.
Rietveld, H. (1969). A profile refinement method for nuclear and magnetic structures. Journal of applied Crystallography, 2(2), 65-71.
Rowse, J., & Jepson, W. (1972). The determination of quartz in clay materials: A critical comparison of methods. Journal of Thermal Analysis and Calorimetry, 4(1), 169-175.
Sheffield, G. S. (1994). Quantitative measurement of crystalline silica by thermal analysis. Analytica chimica acta, 286(1), 125-128.
Weiss, B., Boettner, E. A., & Stenning, M. (1970). Determination of quartz. Archives of Environmental Health: An International Journal, 20(1), 37-44.

## Claims

1. Method for quantifying the phase amounts of a carbonated rock sample comprising the steps of:
calibrating a differential thermal analysis, DTA, line from a plurality of quartz references in varying amounts of quartz relative to the total reference amount, with the amount of quartz as a function of the area of endothermic peak quartz inversion;
obtaining a DTA area of endothermic peak inversion of the quartz in the sample and calculating the amount of quartz present in the sample using the calibrated DTA line;
obtaining an X-ray diffraction, XRD, pattern from the sample;
using Rietveld Refinement to fit XRD patterns of crystalline carbonated rock components to the obtained sample XRD pattern, provided that the amount of quartz present in the sample is fixed to be the previously calculated amount;
calculating the amounts of the crystalline rock components, other than quartz, from the fitted XRD patterns;
calculating a total amount of amorphous and minor phases present in the sample by subtracting the sum of the calculated amounts of the crystalline rock components, including quartz, from the total sample amount.

2. Method according to the previous claim wherein the DTA area of endothermic peak inversion is obtained at a temperature ranging from 40° to 800°C, with a step heating of 10 °C/min.

3. Method according to any of the previous claims wherein the DTA area of endothermic peak inversion is obtained at a temperature of 573°C.

4. Method according to any of the previous claims wherein the DTA area of endothermic peak inversion is obtained under nitrogen atmosphere at a flow rate of 70 mL/min.

5. Method according to any of the previous claims wherein the XRD pattern is obtained at 0.02° steps for 4 seconds or more by step.

6. Method according to any of the previous claims wherein the XRD pattern obtained comprise diffraction peaks from atomic planes with d=17Å to d=1,3Å.

7. Method according to any of the previous claims wherein instrument Rietveld parameters are obtained by fitting corundum (Al₂O₃) reference.

8. Method according to any of the previous claims wherein the Rietveld parameters to fit XRD patterns of crystalline carbonated rock are Instrument/sample alignment instrumental parameters, preferred orientation, grain size and strain-stress characteristics of grains, the crystal structure parameters and the proportions of each mineral phase.

9. Method according to any of the previous claims wherein the calibration of DTA is made in the same conditions of the DTA to obtain the area of endothermic peak inversion of the sample.

10. Method according to any of the previous claims wherein the plurality of quartz references used for calibrating a Differential Thermal Analysis, DTA, comprise a variable amount of calcite euhedral crystals to make up the total reference amount.

11. Method, according to any of the previous claims wherein the variable amount of calcite euhedral crystals in the plurality of quartz references are in steps proportions of 5%.

12. Method, according to any of the previous claims wherein the plurality of quartz references has particles size of 63 µm or less.

13. Method, according to any of the previous claims wherein the carbonated rock sample is ground to obtain particles size of 63 µm or less, in particular wherein the rock sample after ground is quartered for the differential thermal analysis, DTA, and for X-ray diffraction, XRD.

14. Method, according to any of the previous claims wherein the carbonate rock is a marble, a limestone, lime mortar or a marl.

15. Non-transitory storage media comprising computer program instructions for implementing a device for quantifying the phase amounts of a carbonated rock sample, the computer program instructions including instructions which, when executed by a processor, cause the processor to carry out the method of any of the claims 1-14.
